# EUROPEAN PATENT APPLICATION

(11) **EP 4 620 514 A1**
(43) Date of publication of application: **24.09.2025**
(21) Application number: 23892064.9
(22) Date of filing: 17.11.2023
(51) Int. Cl.: A61N 1/36, A61N 1/32, A61N 1/40, A61N 5/06, A61N 5/067, A61B 18/20, A61B 90/00, A61B 18/00

(54) **COSMETIC PROCEDURE DEVICE**

(30) Priority: 18.11.2022 KR 20220155216
(71) Applicant: APR CO., LTD., Seoul 05551 (KR)
(72) Inventor: JI, Jong Chul, Seoul 08592 (KR); LEE, Gyoun Jung, Seoul 08592 (KR); PARK, Seung Woo, Seoul 08592 (KR)
(74) Representative: SONN Patentanwälte GmbH & Co KG
(86) International application number: PCT/KR2023/018537
(87) International publication number: WO 2024/107001

(57) **Abstract**

Disclosed is a cosmetic procedure device. A cosmetic procedure device according to an embodiment of the present invention is intended to carry out a cosmetic procedure on the user's skin and may comprise: a main body; a plurality of direction indication units arranged on the main body to display different directions, respectively; a displacement detection unit arranged in the main body to detect a displacement of the main body during the cosmetic procedure; and a control unit arranged in the main body to control the plurality of direction indication units to display at least one of a direction in which the main body can proceed during the cosmetic procedure and a direction in which the main body is proceeding during the cosmetic procedure.

## Description

### TECHNICAL FIELD

The present invention relates to a cosmetic procedure device, and more particularly, to a cosmetic procedure device that performs a cosmetic procedure in contact with the user's skin.

### BACKGROUND

In recent years, home-use cosmetic procedure devices that allow users to perform cosmetic procedures such as massage and hair removal by themselves at home have become increasingly widespread. Home-use cosmetic procedure devices enable users to conveniently perform cosmetic procedures at their desired time in their own homes, thereby lowering the barrier to cosmetic procedures and improving accessibility.

With the increasing adoption of home-use cosmetic procedure devices, the performance of such devices has been significantly improved compared to conventional devices, and the effectiveness of the procedures has also been enhanced. This means that if the user operates the device improperly or fails to use it safely and appropriately, both the likelihood and severity of side effects may increase. Specifically, home-use cosmetic procedure devices perform procedures such as hair removal and massage using laser or high-frequency energy. However, if laser or high-frequency energy is excessively applied to the user's skin during use, side effects such as burns or pigmentation may occur.

In this context, ensuring the safety of home-use cosmetic procedure devices has come to be recognized as an important task, no less significant than enhancing their primary functions.
(Patent Document) Korean Patent Registration No. 1707659

### DISCLOSURE

### TECHNICAL PROBLEM

The present invention is to solve the above problems, and the present invention is directed to providing a cosmetic procedure device that guides safe use by the user during a skin cosmetic procedure.

The problems of the present invention are not limited to those mentioned above, and other problems not mentioned will be clearly understood by those of ordinary skill in the art from the following description.

### TECHNICAL SOLUTION

According to an aspect of the present invention, provided is a cosmetic procedure device for performing a cosmetic procedure on a user's skin, comprising: a main body; a plurality of direction indication units disposed on the main body to indicate different directions, respectively; a displacement detection unit disposed on the main body and configured to detect displacement of the main body during the cosmetic procedure; and a control unit disposed on the main body and configured to control the plurality of direction indication units to indicate one or more of a direction in which the main body can proceed during the cosmetic procedure and a direction in which the main body is proceeding during the cosmetic procedure.

In this case, the control unit may be configured to determine a direction in which the main body can proceed during the cosmetic procedure based on detection information from the displacement detection unit, such that a redundant procedure is not performed on the same area of the skin.

In addition, the control unit may be configured to cumulatively determine displacement of the main body after the start of the cosmetic procedure.

In addition, the control unit may be configured to generate a warning signal when it is determined that the main body is performing a redundant procedure on the same area of the skin.

In addition, the control unit may be configured to cumulatively determine the number of times a redundant procedure is performed on the same area of the skin, and to increase one or more of intensity and frequency of the warning signal as the number of redundant procedures on the same area increases.

In addition, the control unit may be configured to determine whether a redundant procedure is being performed on the same area with relatively high precision when the body part of the user on which the cosmetic procedure is being performed is a first body part, and to determine whether a redundant procedure is being performed on the same area with relatively low precision when the body part is a second body part.

In addition, the first body part may include a face of the user, and the second body part may include one or more of a thigh, an abdomen, and a buttock of the user.

In addition, the control unit may be configured to control the direction indication units to output the warning signal.

In addition, the cosmetic procedure device may further include a warning output unit configured to output the warning signal under control of the control unit.

In addition, the warning signal may include one or more of vibration and sound.

In addition, when an active mode in which the traveling direction of the main body is actively guided is selected, the control unit may be configured to receive a preset path along which the main body is to proceed while avoiding a redundant procedure on the same area of the skin during the cosmetic procedure, and to control the plurality of direction indication units to indicate a direction in which the main body is to proceed during the cosmetic procedure based on the preset path and detection information from the displacement detection unit.

In addition, the cosmetic procedure device may further include a storage unit disposed in the main body and configured to store one or more preset paths in accordance with a predetermined criterion.

In addition, the cosmetic procedure device may further include a communication unit disposed in the main body and configured to receive the preset path from an external terminal.

In addition, when a passive mode in which a direction in which the main body is currently proceeding during the cosmetic procedure is indicated is selected, the control unit may be configured to control the plurality of direction indication units to indicate the direction in which the main body is currently proceeding when the main body moves during the cosmetic procedure.

In addition, each of the plurality of direction indication units may include at least one direction indication light source.

In addition, at least one of the plurality of direction indication units may include a direction indication light source that emits light for indicating a direction toward the skin of the user.

In addition, the plurality of direction indication units may be disposed at least at the front, rear, left, and right sides of the main body, respectively.

### ADVANTAGEOUS EFFECT

According to the above configuration, the cosmetic procedure device according to the present invention can prevent a redundant procedure on the same area of the skin and guide safe use through a direction indication unit that indicates at least one of a direction in which the main body can proceed and a direction in which the main body is proceeding during the cosmetic procedure.

Advantageous effects of the present invention are not limited to the above-described effects and should be understood to include all effects that can be inferred from the configuration of the invention described in the detailed description or claims of the present invention.

### BRIEF DESCRIPTION OF THE DRAWINGS

FIG. 1 is a block diagram of a cosmetic procedure device according to an exemplary embodiment of the present invention.
FIG. 2 is a front-upper perspective view of a cosmetic procedure device according to an exemplary embodiment of the present invention.
FIG. 3 is a rear-lower perspective view of a cosmetic procedure device according to an exemplary embodiment of the present invention.
FIG. 4 is an illustrative view showing a direction indication by a direction indication unit of a cosmetic procedure device according to an exemplary embodiment of the present invention.
FIG. 5 is an illustrative view showing an operation of a cosmetic procedure device in a normal mode according to an exemplary embodiment of the present invention.
FIG. 6 is an illustrative view showing an operation of a cosmetic procedure device in an active mode according to an exemplary embodiment of the present invention.
FIG. 7 is an illustrative view showing an operation of a cosmetic procedure device in a passive mode according to an exemplary embodiment of the present invention.

### MODES OF THE INVENTION

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein. In the drawings, parts unrelated to the description are omitted for clarity of description of the present invention, and throughout the specification, same or similar reference numerals denote same elements.

Terms and words used in the present specification and claims should not be construed as limited to their usual or dictionary definition. They should be interpreted as meaning and concepts consistent with the technical idea of the present invention, based on the principle that inventors may appropriately define the terms and concepts to describe their own invention in the best way.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

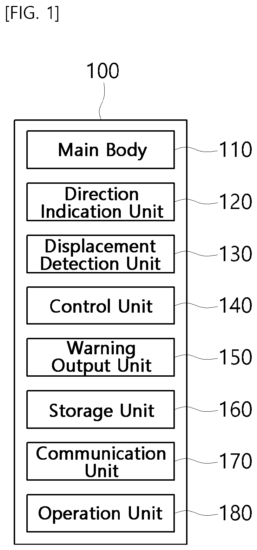

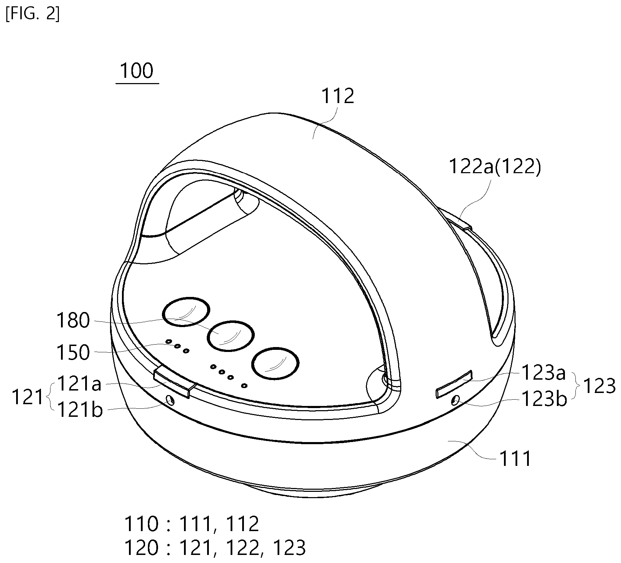

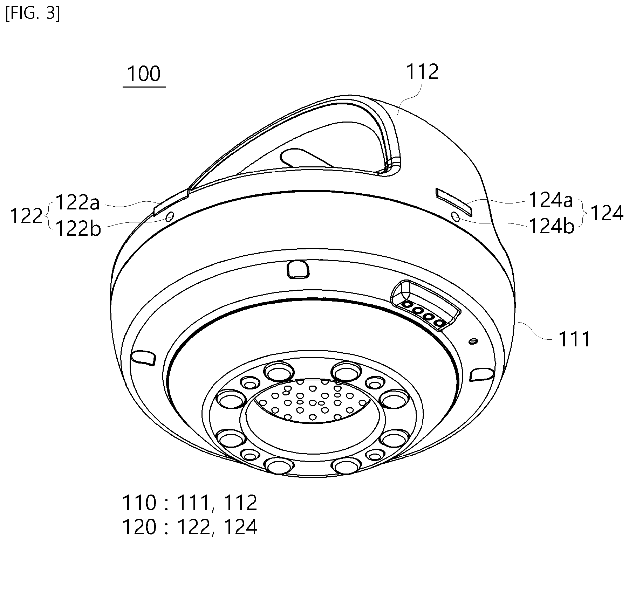

FIG. 1 is a block diagram of a cosmetic procedure device according to an exemplary embodiment of the present invention. In addition, FIG. 2 is a front-upper perspective view of a cosmetic procedure device according to an exemplary embodiment of the present invention, and FIG. 3 is a rear-lower perspective view of a cosmetic procedure device according to an exemplary embodiment of the present invention.

A cosmetic procedure device 100 according to an embodiment of the present invention allows a user to directly perform a cosmetic procedure on their own skin. That is, the user may bring the cosmetic procedure device 100 according to an embodiment of the present invention into contact with their own skin to directly perform a cosmetic procedure. For example, the cosmetic procedure device 100 according to an embodiment of the present invention may perform procedures such as high-frequency massage and laser hair removal. In addition, it may additionally provide a suction function that stimulates the skin by suctioning it.

Referring to FIGS. 1 to 3, the cosmetic procedure device 100, according to an embodiment of the present invention, may include a main body 110, a direction indication unit 120, a displacement detection unit 130, a control unit 140, and a warning output unit 150.

The main body 110 has a shape that allows it to come into contact with a predetermined area of the user's skin. Components for performing a cosmetic procedure may be disposed in the main body 110. For example, components (not shown) for emitting high-frequency energy or lasers may be disposed in the main body 110. In addition, components (not shown) for providing a suction function that stimulates the user's skin by drawing it in may also be disposed in the main body 110.

In an embodiment of the present invention, the main body 110 includes a first portion 111, which is provided such that a lower side thereof comes into contact with the user's skin and in which components for performing a cosmetic procedure are disposed, and a second portion 112, which is connected to an upper side of the first portion 111 and configured to be held by the user. In this case, the first portion 111 may have a cylindrical shape, and the second portion 112 may have an arched shape. When the first portion 111 has a cylindrical shape, the uniformity of contact with the user's skin may be improved. In addition, the user may easily move the main body 110 while holding the second portion 112 and may appropriately press the first portion 111 toward the user's skin.

A plurality of direction indication units 120 are disposed on the main body 110 to indicate different directions, respectively. In an embodiment of the present invention, a plurality of direction indication units 120 may also be respectively disposed at least on the front, rear, left, and right sides of the main body 110. In other words, the plurality of direction indication units 120 may include a first direction indication unit 121 indicating the front direction of the main body 110, a second direction indication unit 122 indicating the rear direction of the main body 110, a third direction indication unit 123 indicating the left direction of the main body 110, and a fourth direction indication unit 124 indicating the right direction of the main body 110.

Each of the plurality of direction indication units 120 may include at least one direction indication light source. In other words, the plurality of direction indication units 120 may indicate directions through light emitted from the light source.

In an embodiment of the present invention, the first direction indication unit 121 may include two direction indication light sources. For example, the first direction indication unit 121 may include a first-1 direction indication light source 121a and a first-2 direction indication light source 121b. More specifically, the first-1 direction indication light source 121a may be disposed at an upper front side of the first portion 111 of the main body 110. In addition, the first-2 direction indication light source 121b may be disposed below the first-1 direction indication light source 121a.

The first-1 direction indication light source 121a may indicate a front portion of the main body 110 through lighting or blinking. In addition, the first-2 direction indication light source 121b may emit light having directivity toward the direction to be indicated. For example, the first-2 direction indication light source 121b may emit linear light toward the front of the main body 110.

Meanwhile, at least one of the plurality of direction indication units 120 may include a direction indication light source that emits light for indicating a direction toward the user's skin. For example, in an embodiment of the present invention, the first-2 direction indication light source 121b may emit light toward the user's skin located on the front side of the main body 110.

In this case, the first-2 direction indication light source 121b may emit light having a predetermined shape (e.g., an arrow shape) toward the user's skin. Through this, the user may visually and clearly recognize the direction in which the main body 110 can proceed or the main body 110 is proceeding.

An embodiment of the present invention includes both the first-1 direction indication light source 121a and the first-2 direction indication light source 121b in the first direction indication unit 121, but this is merely one example. According to a variation of an embodiment of the present invention, the first direction indication unit 121 may include only one of the first-1 direction indication light source 121a and the first-2 direction indication light source 121b.

In an embodiment of the present invention, each of the second to fourth direction indication units 122 to 124 may also include two direction indication light sources, like the first direction indication unit 121. More specifically, the second direction indication unit 122 may include a second-1 direction indication light source 122a, which is disposed at the rear portion of the main body 110 to indicate the rear portion of the main body 110 through lighting or blinking, and a second-2 direction indication light source 122b, which is disposed at the rear portion of the main body 110 and configured to emit linear light directed toward the rear of the main body 110 or light directed toward the user's skin located at the rear side of the main body 110. In addition, the third direction indication unit 123 may include a third-1 direction indication light source 123a, which is disposed at the left portion of the main body 110 to indicate the left portion of the main body 110 through lighting or blinking, and a third-2 direction indication light source 123b, which is disposed at the left portion of the main body 110 and configured to emit linear light directed toward the left of the main body 110 or light directed toward the user's skin located on the left side of the main body 110. The fourth direction indication unit 124 may include a fourth-1 direction indication light source 124a, which is disposed at the right portion of the main body 110 to indicate the right portion of the main body 110 through lighting or blinking, and a fourth-2 direction indication light source 124b, which is disposed at the right portion of the main body 110 and configured to emit linear light directed toward the right of the main body 110 or light directed toward the user's skin located on the right side of the main body 110.

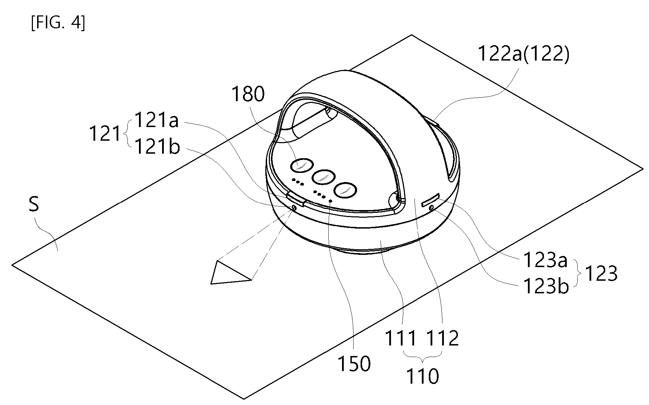

FIG. 4 is an illustrative view showing a direction indication by a direction indication unit of a cosmetic procedure device according to an exemplary embodiment of the present invention.

Referring to FIG. 4, when the direction in which the main body 110 of the cosmetic procedure device 100 according to an exemplary embodiment of the present invention can proceed or is proceeding is the front of the main body 110, the first-1 direction indication light source 121a of the first direction indication unit 121 disposed at the front of the main body 110 may be turned on or blink. In addition, the first-2 direction indication light source 121b may emit light toward the user's skin located at the front side of the main body 110. In this case, the light emitted by the first-2 direction indication light source 121b may have an arrow shape.

The displacement detection unit 130 is disposed in the main body 110 and detects a displacement of the main body 110 during a cosmetic procedure. The displacement detection unit 130 may include one or more of an inertial sensor, a gyro sensor, and an acceleration sensor. The displacement detection unit 130 detects a displacement of the main body 110 after the start of the cosmetic procedure and provides the detected displacement to the control unit 140. Based on the information detected by the displacement detection unit 130, the control unit 140 may determine a path along which the main body 110 has moved after the start of the cosmetic procedure, a relative positional relationship between a cosmetic procedure start position and the current position of the main body 110, and the like.

The control unit 140 is disposed in the main body 110 and controls the plurality of direction indication units 120 to indicate one or more of a direction in which the main body 110 can proceed during a cosmetic procedure and a direction in which the main body 110 is proceeding during the cosmetic procedure. The control unit 140 may include one or more processors, memory elements, and the like capable of performing storage and processing of data.

For example, when the direction indication unit 120 includes direction indication light sources, the control unit 140 may perform control such that the direction indication light source indicating a direction to be displayed is turned on or blinks, and the direction indication light sources indicating other directions remain turned off. Accordingly, the user may recognize a direction in which the main body 110 can proceed or is proceeding through the light emitted by the light source.

The control unit 140 may determine a direction in which the main body 110 can proceed based on the detection information from the displacement detection unit 130, so that the main body 110 does not perform a redundant procedure on the same area of the skin (S) during the cosmetic procedure. As described above, the cosmetic procedure device 100 according to an embodiment of the present invention may emit high-frequency energy, laser, or the like onto the user's skin (S). However, if high-frequency energy, laser, or the like is excessively applied to the same area of the skin (S), side effects such as burns or pigmentation may occur. To prevent such side effects, the control unit 140 may determine, as a direction in which the main body 110 can proceed, a direction that avoids a redundant procedure on the same area of the skin (S) during the cosmetic procedure. The control unit 140 controls the direction indication unit 120 to inform the user of the direction in which the main body 110 can proceed, as determined in this manner.

In this regard, the control unit 140 may cumulatively determine the displacement of the main body 110 after the start of the cosmetic procedure. The control unit 140 may calculate a relative position between a start position of the cosmetic procedure and a current position of the main body 110 using the detection information from the displacement detection unit 130, and based on this, may determine a direction in which the main body 110 moves toward an area of the skin (S) where the cosmetic procedure has already been performed as a direction in which the main body 110 cannot proceed, and may determine a direction in which the main body 110 can avoid a redundant procedure on the same area of the skin (S) as a direction in which the main body 110 can proceed.

The control unit 140 may generate a warning signal when it is determined that the main body 110 is performing a redundant procedure on the same area of the user's skin (S). The warning signal may be generated in the form of a control signal for the direction indication unit 120 or a control signal for the warning output unit 150, which will be described later.

In this case, when the warning signal is generated in the form of a control signal for the direction indication unit 120, the control unit 140 may control the direction indication unit 120 to output the warning signal. For example, the control unit 140 may control the direction indication unit 120 such that all light sources included in the direction indication unit 120 blink at a predetermined cycle according to the warning signal.

The control unit 140 may cumulatively determine the number of redundant procedures performed on the same area of the skin (S), and as the number of redundant procedures on the same area increases, may increase one or more of the intensity and frequency of the warning signal. For example, when the control unit 140 determines that the user has performed a redundant procedure twice on a predetermined area of the skin (S) during the cosmetic procedure, it may generate a warning signal with relatively low intensity or frequency, and when it determines that a redundant procedure has been performed three or more times on the predetermined area, it may generate a warning signal with higher intensity or frequency.

The control unit 140 may determine whether a redundant procedure is being performed on the same area with relatively high precision when the body part of the user undergoing the cosmetic procedure is a first body part, and may determine whether a redundant procedure is being performed on the same area with relatively low precision when the body part is a second body part. For example, the first body part may include the user's face, and the second body part may include one or more of the user's thigh, abdomen, and buttocks.

The user's face is an area where the skin is thin, and if scarring occurs due to burns or the like, it can have a significant impact on daily life. As such, a relatively sensitive body part of the user may be classified as the first body part, and when the cosmetic procedure is performed on the first body part, the control unit 140 may determine whether a redundant procedure is being performed with relatively high precision and accurately provide a warning regarding the redundant procedure, thereby minimizing side effects.

In comparison, areas such as the user's thighs and buttocks have relatively thicker skin, and in the event of side effects, the impact on daily life is relatively minor. As such, a relatively less sensitive body part of the user may be classified as the second body part, and when the cosmetic procedure is performed on the second body part, the control unit 140 may determine whether a redundant procedure is being performed with relatively low precision, such that a warning for the redundant procedure is provided in a relatively less strict manner, and the cosmetic procedure can be performed as much as possible along the path desired by the user.

Meanwhile, the control unit 140 may determine a direction in which the main body 110 is being moved by the user during the cosmetic procedure using the detection information from the displacement detection unit 130, and may control the direction indication unit 120 to inform the user of the direction in which the main body 110 is proceeding. According to such control by the control unit 140, the direction indication unit corresponding to the direction in which the main body 110 is moving displays the direction, and the user can receive feedback on the movement direction of the main body 110.

The warning output unit 150 outputs the warning signal under the control of the control unit 140. As described above, the warning signal generated by the control unit 140 may be formed in the form of a control signal for the direction indication unit 120, but it may also be generated in other forms. For example, the warning signal may include one or more of vibration and sound. The warning output unit 150 may output a warning signal in such a form.

Referring to FIG. 2, the warning output unit 150 may be a speaker. In this regard, the warning signal may be in the form of an audible sound or a voice notification. Meanwhile, the warning signal may be in the form of vibration, and in this case, the warning output unit 150 may be a vibrator.

Hereinafter, an example of the operation of the cosmetic procedure device 100 according to an embodiment of the present invention will be described in detail.

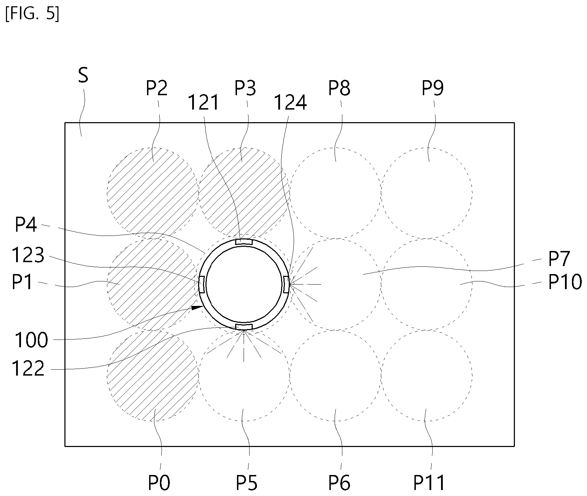

FIG. 5 is an illustrative view showing an operation of a cosmetic procedure device in a normal mode according to an exemplary embodiment of the present invention.

Referring to FIG. 5, the cosmetic procedure device 100 according to an embodiment of the present invention is performing a cosmetic procedure on the user's skin (S). The cosmetic procedure device 100 is currently located at a fourth position (P4) on the user's skin (S) after moving from a procedure start position (P0) through a first position (P1), a second position (P2), and a third position (P3). At the fourth position (P4), the control unit 140 determines the next direction in which the cosmetic procedure device 100 can proceed.

At the fourth position (P4), based on the orientation of the main body 110 of the cosmetic procedure device 100, the front side and the left side of the main body 110 are areas where the cosmetic procedure has already been performed. If the cosmetic procedure device 100 moves forward or to the left of the main body 110 from the fourth position (P4), a redundant procedure will be performed on the first position (P1) or the third position (P3). Therefore, at the fourth position (P4), a direction in which the main body 110 can proceed after the cosmetic procedure may be determined as the rear or right side of the main body 110. The control unit 140 may, based on such a determination, control the second direction indication unit 122, which indicates the rear of the main body 110, and the fourth direction indication unit 124, which indicates the right side of the main body 110, to inform the user to proceed with the cosmetic procedure device 100 to the rear or the right side of the main body 110.

Meanwhile, when the cosmetic procedure device 100 is moved by the user in the forward or leftward direction of the main body 110 at the fourth position (P4), the control unit 140 may generate a warning signal. The generated warning signal may be output through the direction indication unit 120 or the warning output unit 150.

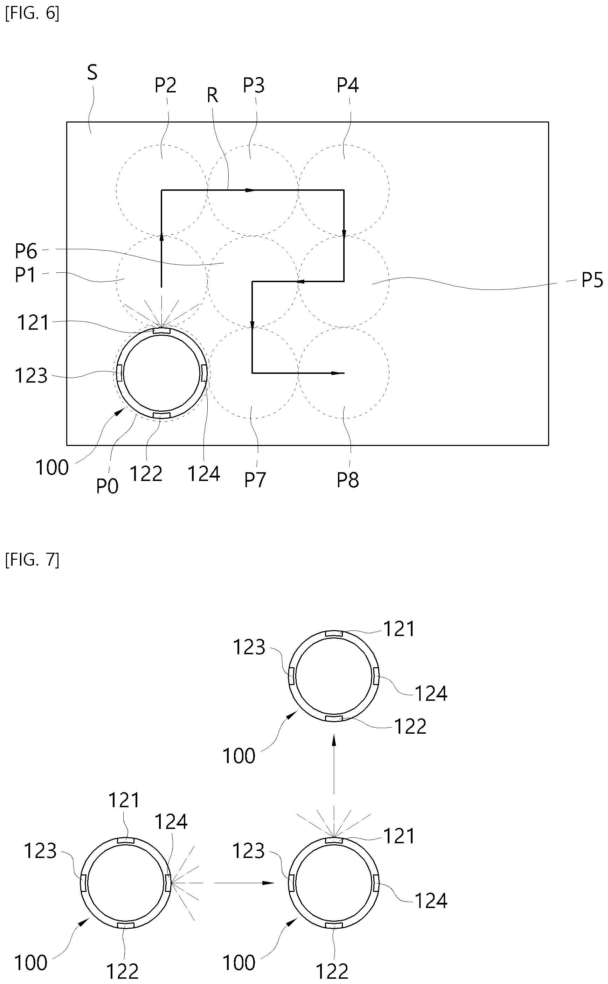

FIG. 6 is an illustrative view showing an operation of a cosmetic procedure device in an active mode according to an exemplary embodiment of the present invention. Here, the active mode refers to a mode in which the traveling direction of the main body 110 is actively guided during the cosmetic procedure.

In the active mode, the control unit 140 may receive a preset path along which the main body 110 is to proceed during the cosmetic procedure while avoiding redundant procedures on the same area of the user's skin (S), and may control the plurality of direction indication units 120 to indicate the direction in which the main body 110 is to proceed during the cosmetic procedure, based on the preset path and detection information from the displacement detection unit 130.

Referring to FIG. 6, a preset path (R) preset for performing a cosmetic procedure on a predetermined area of the user's skin (S) is set to reach an eighth position (P8) via a first position (P1) to a seventh position (P7), starting from a cosmetic procedure start position (P0). The control unit 140 controls the plurality of direction indication units 120 to guide a movement path of the cosmetic procedure device 100 during the cosmetic procedure based on the preset path (R).

First, after the cosmetic procedure is performed at the cosmetic procedure start position (P0), the control unit 140 controls the first direction indication unit 121, which is disposed at the front of the main body 110, to indicate the forward direction of the main body 110, so that movement toward the first position (P1) located at the front side of the main body 110 is guided.

Next, after the cosmetic procedure is performed at the first position (P1), the control unit 140 controls the first direction indication unit 121, which is disposed at the front of the main body 110, to indicate the forward direction of the main body 110, so that movement toward the second position (P2) located at the front side of the main body 110 is guided.

In addition, after the cosmetic procedure is performed at the second position (P2), the control unit 140 controls the fourth direction indication unit 124, which is disposed at the right portion of the main body 110, to indicate the rightward direction of the main body 110, so that movement toward the third position (P3) located on the right side of the main body 110 is guided.

In addition, after the cosmetic procedure is performed at the third position (P3), the control unit 140 controls the fourth direction indication unit 124, which is disposed at the right portion of the main body 110, to indicate the rightward direction of the main body 110, so that movement toward the fourth position (P4) located on the right side of the main body 110 is guided.

In addition, after the cosmetic procedure is performed at the fourth position (P4), the control unit 140 controls the second direction indication unit 122, which is disposed at the rear portion of the main body 110, to indicate the rearward direction of the main body 110, so that movement toward the fifth position (P5) located at the rear of the main body 110 is guided.

In addition, after the cosmetic procedure is performed at the fifth position (P5), the control unit 140 controls the third direction indication unit 123, which is disposed at the left portion of the main body 110, to indicate the leftward direction of the main body 110, so that movement toward the sixth position (P6) located on the left side of the main body 110 is guided.

In addition, after the cosmetic procedure is performed at the sixth position (P6), the control unit 140 controls the second direction indication unit 122, which is disposed at the rear portion of the main body 110, to indicate the rearward direction of the main body 110, so that movement toward the seventh position (P7) located at the rear of the main body 110 is guided.

Finally, after the cosmetic procedure is performed at the seventh position (P7), the control unit 140 controls the fourth direction indication unit 124, which is disposed at the right portion of the main body 110, to indicate the rightward direction of the main body 110, so that movement toward the eighth position (P8) located on the right side of the main body 110 is guided.

As such, by guiding the traveling direction of the cosmetic procedure device 100 along the preset path (R), the control unit 140 enables the user to safely perform the cosmetic procedure while avoiding a redundant procedure on the same area of the skin (S).

In the active mode, when the cosmetic procedure device 100 deviates from the guidance based on the preset path (R) and moves in a direction to perform a redundant procedure on the same area of the user's skin (S), the control unit 140 may generate a warning signal. The generated warning signal may be output through one or more of the direction indication unit 120 and the warning output unit 150.

Meanwhile, in connection with the active mode as described above, the cosmetic procedure device 100 according to an embodiment of the present invention may further include a storage unit 160, which is disposed in the main body 110 and stores one or more preset paths in accordance with a predetermined criterion. The storage unit 160 may be implemented with various memory devices that store one or more preset paths.

In addition, the cosmetic procedure device 100 according to an embodiment of the present invention may further include a communication unit 170, which is disposed in the main body 110 and receives the preset path from an external terminal. For example, the communication unit 170 may receive the preset path from a mobile terminal such as a smartphone through a short-range wireless communication method.

In an embodiment of the present invention, both the storage unit 160 and the communication unit 170 may be provided, or only one of them may be provided.

FIG. 7 is an illustrative view showing an operation of a cosmetic procedure device in a passive mode according to an exemplary embodiment of the present invention. Here, the passive mode refers to a mode that provides simple feedback on the direction in which the main body 110 is currently proceeding during the cosmetic procedure.

Referring to FIG. 7, in the passive mode, when the cosmetic procedure device 100 is moving to the right side of the main body 110, the control unit 140 controls the fourth direction indication unit 124, which is disposed at the right portion of the main body 110, to inform the user that the cosmetic procedure device 100 is moving in the rightward direction of the main body 110. In addition, when the cosmetic procedure device 100 is moving in the forward direction of the main body 110, the control unit 140 controls the first direction indication unit 121, which is disposed at the front portion of the main body 110, to inform the user that the cosmetic procedure device 100 is moving in the forward direction of the main body 110.

Meanwhile, the cosmetic procedure device 100 according to an embodiment of the present invention may further include an operation unit 180 configured to receive operation information for power on/off, selection of various modes, and the like. Referring to FIG. 2, the operation unit 180 may include one or more buttons. Of course, this is merely an example, and the operation unit 180 may be provided in various forms.

In addition, when the cosmetic procedure device 100 according to an embodiment of the present invention includes the communication unit 170, information for operating the cosmetic procedure device 100 may be transmitted from an external terminal to the communication unit 170, and the control unit 140 may process the information after it is received by the communication unit 170.

Although exemplary embodiments of the present invention have been described, the spirit of the present invention is not limited to the embodiments set forth herein. Those of ordinary skill in the art who understand the spirit of the present invention may easily propose other embodiments through supplement, change, removal, addition, etc. of elements within the same idea, but the embodiments will also be within the scope of the present invention.

## Claims

1. A cosmetic procedure device for performing a cosmetic procedure on a user's skin, comprising:
a main body;
a plurality of direction indication units disposed on the main body to indicate different directions, respectively;
a displacement detection unit disposed on the main body and configured to detect displacement of the main body during the cosmetic procedure; and
a control unit disposed on the main body and configured to control the plurality of direction indication units to indicate one or more of a direction in which the main body can proceed during the cosmetic procedure and a direction in which the main body is proceeding during the cosmetic procedure.

2. The cosmetic procedure device of claim 1, wherein the control unit is configured to determine a direction in which the main body can proceed during the cosmetic procedure based on detection information from the displacement detection unit, such that a redundant procedure is not performed on the same area of the skin.

3. The cosmetic procedure device of claim 2, wherein the control unit is configured to cumulatively determine displacement of the main body after the start of the cosmetic procedure.

4. The cosmetic procedure device of claim 2, wherein the control unit is configured to generate a warning signal when it is determined that the main body is performing a redundant procedure on the same area of the skin.

5. The cosmetic procedure device of claim 4, wherein the control unit is configured to cumulatively determine the number of times a redundant procedure is performed on the same area of the skin, and to increase one or more of intensity and frequency of the warning signal as the number of redundant procedures on the same area increases.

6. The cosmetic procedure device of claim 4, wherein the control unit is configured to determine whether a redundant procedure is being performed on the same area with relatively high precision when the body part of the user on which the cosmetic procedure is being performed is a first body part, and to determine whether a redundant procedure is being performed on the same area with relatively low precision when the body part is a second body part.

7. The cosmetic procedure device of claim 6, wherein the first body part comprises a face of the user, and the second body part comprises one or more of a thigh, an abdomen, and a buttock of the user.

8. The cosmetic procedure device of claim 4, wherein the control unit is configured to control the plurality of direction indication units to output the warning signal.

9. The cosmetic procedure device of claim 4, further comprising a warning output unit configured to output the warning signal under control of the control unit.

10. The cosmetic procedure device of claim 9, wherein the warning signal comprises one or more of vibration and sound.

11. The cosmetic procedure device of claim 1, wherein, when an active mode in which the traveling direction of the main body is actively guided is selected, the control unit is configured to receive a preset path along which the main body is to proceed while avoiding a redundant procedure on the same area of the skin during the cosmetic procedure, and to control the plurality of direction indication units to indicate a direction in which the main body is to proceed during the cosmetic procedure based on the preset path and detection information from the displacement detection unit.

12. The cosmetic procedure device of claim 11, further comprising a storage unit disposed in the main body and configured to store one or more preset paths in accordance with a predetermined criterion.

13. The cosmetic procedure device of claim 11, further comprising a communication unit disposed in the main body and configured to receive the preset path from an external terminal.

14. The cosmetic procedure device of claim 1, wherein, when a passive mode in which a direction in which the main body is currently proceeding during the cosmetic procedure is indicated is selected, the control unit is configured to control the plurality of direction indication units to indicate the direction in which the main body is currently proceeding when the main body moves during the cosmetic procedure.

15. The cosmetic procedure device of claim 1, wherein each of the plurality of direction indication units comprises at least one direction indication light source.

16. The cosmetic procedure device of claim 15, wherein at least one of the plurality of direction indication units comprises a direction indication light source that emits light for indicating a direction toward the skin of the user.

17. The cosmetic procedure device of claim 1, wherein the plurality of direction indication units are disposed at least at the front, rear, left, and right sides of the main body, respectively.
